# EUROPEAN PATENT SPECIFICATION

(11) **EP 3 658 159 B1**
(45) Date of publication and mention of the grant of the patent: **06.07.2022**
(21) Application number: 18768972.4
(22) Date of filing: 25.07.2018
(51) Int. Cl.: A61K 31/714, A61K 33/26, A61K 45/00, A61P 3/02

(54) **PHARMACEUTICAL COMPOSITION FOR USE IN THE TREATMENT OR PREVENTION OF VITAMIN DEFICIENCY AND MINERAL DEFICIENCY IN PATIENTS WHO HAVE BEEN SUBJECTED TO GASTRIC SLEEVE SURGERY**
PHARMAZEUTISCHE ZUSAMMENSETZUNG ZUR VERWENDUNG BEI DER BEHANDLUNG ODER PRÄVENTION VON VITAMINMANGEL UND MINERALIENMANGEL BEI PATIENTEN, DIE EINER MAGENSCHLAUCHOPERATION UNTERZOGEN WURDEN
COMPOSITION PHARMACEUTIQUE DESTINÉE À ÊTRE UTILISÉE DANS LE TRAITEMENT OU LA PRÉVENTION DE CARENCES EN VITAMINES ET MINÉRAUX CHEZ DES PATIENTS AYANT ÉTÉ SOUMIS À UNE GASTRECTOMIE

(30) Priority: 26.07.2017 NL 2019348
(43) Date of publication of application: 03.06.2020
(73) Proprietor: AMS Advanced Medical Supplements B.V., 3014 DA Rotterdam (NL)
(72) Inventor: VAN BERKEL, Sjoerd Arie Pieter, 2451 AT Leimuiden (NL)
(74) Representative: Nederlandsch Octrooibureau
(86) International application number: PCT/NL2018/050517
(87) International publication number: WO 2019/022602

(56) References cited:
- EP-A1- 3 009 133
- WO-A1-2009/153065
- WO-A2-2007/021504
- WO-A2-2009/153064
- BR-A2- PI1 101 592
- FR-A1- 3 031 902
- GB-A- 2 197 587
- US-A1- 2007 031 486
- DATABASE WPI Week 201079 Thomson Scientific, London, GB; AN 2010-P72336 XP002778264, & CN 101 869 129 A (LI G) 27 October 2010 (2010-10-27)
- Andrew Van Osdol ET AL: "MICRONUTRIENT DEFICIENCIES IN PATIENTS AFTER LAPAROSCOPIC ROUX-EN-Y GASTRIC BYPASS VERSUS SLEEVE GASTRECTOMY - Abstract A5082", Surgery for Obesity and Related Diseases, 1 January 2015 (2015-01-01), page S98, XP055451945, Retrieved from the Internet: URL:http://www.soard.org/article/S1550-728 9(15)00445-1/pdf [retrieved on 2018-02-16]

## Description

### TECHNICAL FIELD OF THE INVENTION

The invention relates to a pharmaceutical composition for use in the treatment or prevention of vitamin and mineral deficiencies in patients which have been subjected to gastric sleeve surgery. The present invention further relates to a pharmaceutical composition as such.

### BACKGROUND OF THE INVENTION

Bariatric surgery was first introduced in the 1960's in the United States as a treatment for morbid obesity. In the last decade the number of bariatric procedures has grown rapidly. This increase is largely due to the increase in the number of people suffering from morbid obesity. Patients suffering from morbid obesity have an increased risk for many different diseases, such as type II diabetes, coronary disease, hyperlipidemia, hypertension, sleep apnea, osteoarthritis, several types of cancer and liver steatosis. Furthermore, morbid obesity is also associated, as its name already suggests, with a shortened life expectancy and a reduced quality of life.

Dietary advice, encouraging patients to do more physical exercise and drug therapy are often not effective enough in the treatment of morbid obesity. Hence, surgical procedures are nowadays commonly used to treat morbid obesity. For example, the U.S. National Institutes of Health recommends bariatric surgery for obese people with a body mass index (BMI) of at least 40, and for people with BMI of at least 35 and serious coexisting medical conditions such as diabetes. Accumulating research results provide insight that bariatric surgery is even already appropriate for those with a BMI of 35 to 40 with no comorbidities or a BMI of 30 to 35 with significant comorbidities, and in addition research results indicate that any patient with a BMI of more than 30 with comorbidities could benefit from undergoing bariatric surgery.

In general, bariatric surgery is divided in restrictive techniques and in a combination of restrictive and mal-absorptive techniques. The gastric band and the gastric sleeve are examples of restrictive bariatric surgery. Gastric sleeve surgery and gastric-band surgery provide an average excess weight loss of 30-50%. The Roux-en-Y gastric bypass (hereinafter: RYGB), the duodenal switch and the Scopinaro are examples of combined techniques. The RYGB is the most commonly used technique. Although this technique has a higher risk for complications, it gives a greater excess weight loss, *i.e.* between 50% and 90%. Long-term studies show the procedures altogether cause significant long-term loss of weight, recovery from diabetes, improvement in cardiovascular risk factors, and a mortality reduction from 40% to 23%.

The gastric sleeve procedure was initially designed as a first step in a procedure for the super morbid obese patients named the duodenal switch. It was often performed in two separate procedures, in which the first was the gastric sleeve. When patients had lost a serious amount of weight, additional weight loss was achieved by switching the duodenum in the second procedure often performed a few years after the first step. With only a small percentage of patients undergoing this procedure because of its downsides and high complication rates it took some years to find that many patients had sufficient weight loss with just the first step; the gastric sleeve. Around the year 2000 surgeons started using the gastric sleeve as a stand-alone procedure. Nowadays in general, the relatively lighter patients, *i.e.* patients with a body mass index of less than 40 kg per square meter or younger patients, are often treated by applying a gastric sleeve. The relatively heavier group of patients is most often treated by using a RYGB. However, there are also bariatric centers that only perform gastric sleeves and use the procedure to treat all categories of morbid obese patients.

The gastric sleeve surgery, or 'sleeve gastrectomy', is in general performed as follows. During gastric sleeve surgery the stomach is reduced to about 15% to 30% of its original size. The ideal approximate remaining size of the stomach after the procedure is about 150 mL. After the grater curvature of the stomach is released a longitudinal resection of the stomach is performed using a linear stapler starting from the antrum at the point 5-6 cm from the pylorus and finishing at the fundus close to the cardia. The procedure is performed most often laparoscopically and is irreversible but leads to a tremendous decreased risk of adverse side-effects related to weight loss surgery for specific groups of patients. Most patients subjected to gastric sleeve surgery lose 30% to 50% of their excess body weight in a period of 6-12 months with the sleeve gastrectomy alone. In this regard reference is also made to the article of Rosenthal, R.J. et al., International Sleeve Gastrectomy Expert Panel Consensus Statement: best practice guidelines based on experience of > 12,000 cases, in Surgery for Obesity and Related Diseases 8 (2012) 8-19.

The stomach remains functioning normally after the surgical procedure, so most food stuff can be consumed in small amounts due to its restriction. By applying gastric sleeve surgery, the portion of the stomach is removed which produces the hormone that stimulates hunger (*Ghrelin*)*.* Since the pylorus is preserved during gastric sleeve surgery, it is less likely that patients who underwent said surgery will suffer from dumping syndrome, which is one of the various side effects occurring after applying combined techniques like the RYGB. The chance for the patient who underwent gastric sleeve surgery to develop an ulcer, is relatively small. With gastric sleeve surgery, creating an intestinal bypass is avoided, thereby reducing the risk for the patient to develop intestinal obstruction (blockage due to internal herniation), anemia, osteoporosis and protein deficiency. Gastric sleeve surgery has proven to be very effective as a first stage procedure for high BMI patients (BMI >55 kg per square meter). The gastric sleeve surgical procedure is for example sometimes also used in patients suffering from diseases and impaired health conditions such as anemia due to stomach ulcers, Crohn's disease, irritable bowel syndrome, and many other diseases, which diseases and health issues exclude the possibility to apply intestinal bypass procedures.

Gastric bypass surgery and gastric sleeve surgery are both permanent bariatric surgery methods. Said two types of operations reduce hunger and lead to the highest percentage of weight loss amongst the bariatric surgery methods. However, the procedures and effects thereof on the stomach are different and lead to different adverse effects.

For instance, due to the limitation of food intake, the patients which have been subjected to gastric-bypass surgery find it difficult to keep nutrients levels such as vitamins and minerals at the same level as before said surgical procedure was carried out. Furthermore, many patients which have a gastric bypass have difficulties eating certain foods containing essential nutrients, such as for example red meat. Clinical studies have shown that a high number of patients avoid certain foods as such food products often cause nausea and cause these patients to vomit. Without wishing to be bound by any theory, it is assumed that this is mainly due to a combination of insufficient chewing of the food and the slow passage of certain food product through the (reduced) stomach. Furthermore, due to the bypassing of a significant part of the stomach gastric acid comes in contact with food at a later stage and *intrinsic factor* is no longer released from the stomach and remains intracellularly.

In order to balance the reduced intake and absorbing of these nutrients, patients which have been subjected to bariatric surgery are prescribed multivitamin preparations comprising the recommended daily allowance of these nutrients for health subjects. However, clinical studies with patients that underwent gastric bypass surgery have shown that the use of these multivitamin preparations did not restore the essential vitamin balance and mineral balance in said patients. The current inventors previously provided for an elegant solution to that problem for RYGB patients by their invention related to a multivitamin preparation comprising amongst others 140 times the Recommended Daily Allowance (RDA) of vitamin B12 and 500% of the RDA of iron. See European patent EP3009133 of the current inventors.

Gastric sleeve surgery has the benefit over RYGB in that the procedure is technically easy and is performed in a shorter time. As said, gastric sleeve surgery differs from RYGB, with the main difference being that with the gastric sleeve surgery procedure 70% or more of the stomach is removed, which includes the part of the stomach where the hunger hormone ghrelin is produced. Reduced production of ghrelin provides the beneficial effect of a reduced feeling of hunger for the patient who underwent gastric sleeve surgery. RYGB patients are highly prone to vitamin- and mineral deficiencies in the first postoperative year, but after gastric sleeve surgery the total amounts of gastric acid and intrinsic factor produced are seriously reduced. Although the risk for dangerous vitamin- and mineral deficiencies appears to be lower for patients who underwent gastric sleeve surgery per year, still said risk is apparent to a certain extent. However, it is currently unknown which (doses of) vitamins and minerals could safely be provided to gastric sleeve patients such that these patients have a diminished risk for vitamin and mineral deficiencies while at the same time no new health risk is introduced by accidentally administering too high doses of one or more vitamins and minerals.

FR 3 031 902 A1 relates to a composition comprising 4-150 microgram vitamin B12 and 30-100 mg iron, suitable for the treatment of vitamin and mineral deficiencies in patients having undergone e.g. longitudinal gastrectomy.

BR PI 1101592-6 A2 relates to a micronutritional composition formulated in amongst others organoleptically-isolated, micro-granulated tablets, dispersed and integrated into cereal bars, diet chocolate bars and candy. The composition is suitable for amongst others patients having undergone sleeve gastrectomy.

Hence, a need remains for a medicament / vitamin composition which is able to better restore the vitamin and mineral balance in patients which have been subjected to gastric sleeve surgery.

### SUMMARY OF THE INVENTION

The invention is defined by the claims. Any subject-matter falling outside the scope of the claims is provided for information purposes only.

A first aspect of the invention relates to a pharmaceutical composition for use in the treatment or prevention of vitamin and mineral deficiencies in a patient who has been subjected to gastric sleeve surgery comprising:
- vitamin B12 or a source thereof;
- iron or a source thereof;
- a pharmaceutically acceptable carrier; and
wherein the patient in need thereof is administered by means of said composition per day between 50 µg and 150 µg of vitamin B12 and, between 20 mg and 30 mg iron and less than 0,1 µg of vitamin K, and
wherein a unit dose of the pharmaceutical composition comprises:
   - between 50 µg and 150 µg of vitamin B12;
   - between 20 mg and 30 mg iron; and
   - a pharmaceutically acceptable carrier;
wherein the pharmaceutical composition is devoid of calcium and is devoid of magnesium.

It is preferred that the pharmaceutical composition for use according to the invention is devoid of vitamin K. Furthermore, it is preferred that the pharmaceutical composition for use according to the invention is devoid of choline.

Preferably, the pharmaceutical composition for use according to the invention is provided as a unit dose of the composition comprising:
- 100 µg of vitamin B12; and
- 28 mg iron.

Moreover, the pharmaceutical composition for use according to the invention is preferably provided as an oral fixed dose combination, wherein said oral fixed dose combination is a solid dosage form, such as a capsule, a tablet or a powder.

Clinical studies carried out by the present inventors showed that patients which have been subjected to gastric sleeve surgery need to be supplement with vitamin B12 and iron exceeding 100% of the RDA in order to meet the desired levels of these nutrients.

Clinical studies have further shown that treatment with common multivitamin compositions comprising about 100% of the RDA of minerals and vitamins, was not sufficient to reach a desired iron and vitamin B12 level in said patients. With the pharmaceutical composition according to the present invention it has now for the first time been made possible to avoid iron and vitamin B12 deficiencies in patients which have been subjected to gastric sleeve surgery. Although, it is theoretically possible to achieve with common multivitamin compositions the recommended vitamin B12 intake, the intake of several multivitamin capsules a day will lead to hypervitaminosis of other vitamins present in said common multivitamin compositions, such as hypervitaminosis of vitamin A, K or B6.

A second aspect of the invention relates to a pharmaceutical composition comprising per unit dose:
- between 50 µg and 150 µg of vitamin B12;
- between 20 mg and 30 mg iron; and
- a pharmaceutically acceptable excipient, wherein the pharmaceutical composition is devoid of vitamin K and wherein the pharmaceutical composition is devoid of magnesium and wherein the composition is devoid of calcium,
wherein a unit dose of said composition further comprises:
- between 400 µg and 2000 µg of vitamin A;
- between 1 mg and 5,5 mg of vitamin B1;
- between 0,5 mg and 4 mg copper;
- between 7 mg and 60 mg zinc; and
- between 150 µg and 1000 µg folic acid.

It is preferred that the pharmaceutical composition according to the invention is devoid of choline.

Particularly preferred is the pharmaceutical composition according to the invention, wherein a unit dose of the composition comprises:
- 100 µg of vitamin B12; and
- 28 mg iron.

### DEFINITIONS

The term '*pharmaceutical composition*' as used herein has its conventional meaning and refers to a composition which is pharmaceutically acceptable.

The term '*pharmaceutically acceptable*' as used herein has its conventional meaning and refers to compounds, material, compositions and/or dosage forms, which are, within the scope of sound medical judgment suitable for contact with the tissues of mammals, especially humans, without excessive toxicity, irritation, allergic response and other problem complications commensurate with a reasonable benefit/risk ratio.

The term '*excipient*' as used herein has its conventional meaning and refers to a pharmaceutically acceptable ingredient, which is commonly used in the pharmaceutical technology for preparing a granulate, solid or liquid oral dosage formulation.

The term '*treatment*' as used herein has its conventional meaning and refers to curative, palliative and prophylactic treatment.

The term '*unit dose*' has its conventional meaning and refers to a dosage form which has the capacity of being administered as such to a subject, preferably a human, to be effective, and which can be readily handled and packaged, remaining as a physically and chemically stable unit dose comprising the therapeutic agent. Typical examples of unit doses are tablets and capsules.

The term '*fixed dose combination*' as used herein has its conventional meaning and refers to a combination of defined doses of two or more mineral or vitamins presented in a single unit dose (*e.g.* a tablet or a capsule) and administered as such.

The term '*vitamin B11*' as used herein has its conventional meaning and refers to folic acid.

The term '*gastric sleeve surgery*' as used herein has its conventional meaning and refers to laparoscopic sleeve gastrectomy (LSG). In this regard reference is also made to the article of Rosenthal, R.J. et al., International Sleeve Gastrectomy Expert Panel Consensus Statement: best practice guidelines based on experience of > 12,000 cases, in Surgery for Obesity and Related Diseases 8 (2012) 8-19.

### DETAILED DESCRIPTION OF THE INVENTION

The invention is defined by the claims. Any subject-matter falling outside the scope of the claims is provided for information purposes only.

Besides the great benefits of bariatric surgery, there are also disadvantages for the patients such as hampered food intake, dumping, defecation problems and nutrient deficiencies resulting from malabsorption. Post-operatively, bariatric surgery patients are at increased risk of developing nutrient deficiencies because of vomiting, decreased food intake, food intolerance, reduction of gastric secretions, and strongly reduced absorption surface areas. The latter is perhaps the most important factor in causing nutritional deficiencies.

Furthermore, since patients having a gastric sleeve have a reduced feeling of hunger due to reduced production of for example the hunger hormone ghrelin, there is a tendency of reduced food intake amongst those patients, contributing to a risk for mineral deficiencies and vitamin deficiencies. Indeed, common deficiencies, found in patients which have been subjected to a gastric sleeve surgical procedure, include iron deficiency and vitamin B12 deficiency. The deficiencies that occur after surgery are mostly the result of an inadequate intake and malabsorption of essential micronutrients.

In an attempt to balance the iron deficiency and vitamin B12 deficiency in patients which have been subjected to a gastric sleeve surgery, these patients are prescribed standard multivitamin supplements, which supplements generally comprise 100% of the recommended daily allowance (RDA) for iron, vitamin B12 and other vitamins. However, in the clinical study carried out by the present inventors it has now been shown that the administration of such standard multivitamin preparations did not result in solving the iron deficiency and vitamin B12 deficiency commonly observed in these types of patients who underwent gastric sleeve surgery.

The pharmaceutical composition according to the present invention aims to solve these problems.

A first aspect of the current invention is a pharmaceutical composition for use in the treatment or prevention of vitamin and mineral deficiencies in a patient who has been subjected to gastric sleeve surgery comprising:
- vitamin B12 or a source thereof;
- iron or a source thereof;
- a pharmaceutically acceptable carrier; and
wherein the patient in need thereof is administered by means of said composition per day between about 50 µg and 150 µg of vitamin B12 and between about 20 mg and 30 mg iron and less than 0,1 µg of vitamin K, and
wherein a unit dose of the pharmaceutical composition comprises:
- between 50 µg and 150 µg of vitamin B12;
- between 20 mg and 30 mg iron; and
- a pharmaceutically acceptable carrier;
wherein the pharmaceutical composition is devoid of calcium and is devoid of magnesium. Said composition may comprise between 150 µg and 1000 µg folic acid, preferably between 400 and 600 µg.

It is preferred that the pharmaceutical composition for use according to the invention is devoid of vitamin K. Furthermore, it is preferred that the pharmaceutical composition for use according to the invention is devoid of choline.

Preferably, the pharmaceutical composition for use according to the invention is provided as a unit dose of the composition comprising:
- 100 µg of vitamin B12; and
- 28 mg iron.

The inventors of the present invention remarkably found that in order to avoid iron and vitamin B12 deficiencies in patients that have been subjected to gastric sleeve surgery, increased doses of said nutrients need to be administered, compared to the doses related to about 100% of the RDA commonly provided with regular multivitamin capsules, though surprisingly, at the same time said doses of iron and vitamin B12 should be far lower than the high amounts required to compensate deficiencies in patients that for example underwent RYGB. The administration of standard multivitamin preparations, which generally comprise about 100% of the RDA of said nutrients resulted in an average decline of vitamin B12 and iron in said patients. The administration of the pharmaceutical composition according to the present invention, comprising about 1.250% to about 8.750% of the RDA for vitamin B12 such as about 4.000% of the RDA or about 3.750% of the RDA for vitamin B12 and about 100% to about 600% of the RDA for iron, such as about 200% or about 300% of the for iron resulted in a marked increase of vitamin B12 and a marked increase in iron level within said patients.

This is surprising for reasons that it was generally assumed in the scientific literature that the addition of higher amounts of vitamin B12 and iron would not be helpful due to the fact that after a gastric sleeve surgery the release of intrinsic factor is significantly reduced, which significantly compromises vitamin B12 absorption and iron absorption. Furthermore, also due to the reduced secretion of hydrochloric acid, pepsin and pancreatic enzymes it was assumed that increasing the amounts of vitamin B12 and iron would not have a beneficial effect.

Contrary to what was expected in the art, the present inventors now found that moderately increased doses of said nutrients avoid iron and B12 deficiencies in patients which have been subjected to a gastric sleeve surgical procedure, when said doses are compared to common multivitamin preparations comprising about 100% of the RDA for iron and vitamin B12. The higher doses for vitamin B12 and iron, as for instance administered to Roux and Y gastric bypass (RYGB) patients, are not recommendable nor required for the purpose of increasing blood serum levels of vitamin B12 and iron in gastric sleeve surgery patients, since such high doses of vitamin B12 and iron bear a risk for adverse side effects such as occurrence of hyper-vitaminosis.

Although it is theoretically possible to achieve with common multivitamin compositions the recommended vitamin B12 intake with regard to serum levels in the gastric sleeve surgery patient, the intake of several multivitamin capsules a day will lead to hyper-vitaminosis of other vitamins present in said common multivitamin compositions, such as hyper-vitaminosis of vitamin A, vitamin B1 and/or vitamin B6. To further lower the risk for hyper-vitaminosis of vitamin B6, a unit dose of the pharmaceutical composition for use of the invention preferably comprises less than 3 mg vitamin B6, and about 2 mg vitamin B6 per unit dose is typically applied.

In this regard it is further noted that the administration of standard multivitamin preparations comprising about 100% of the RDA for vitamin A already resulted in hyper-vitaminosis of said vitamin in patients which have been subjected to a gastric sleeve surgical procedure.

In an even further preferred embodiment of the present invention, the pharmaceutical composition for use according to the invention is a composition wherein a unit dose of the composition comprises:
- between 50 µg and 150 µg of vitamin B12, preferably 100 µg of vitamin B12;
- between 20 mg and 30 mg iron, preferably 28 mg iron; and
- a pharmaceutically acceptable carrier.

Thus, even more preferred is the pharmaceutical composition for use according to the invention wherein a unit dose of the composition comprises:
- 100 µg of vitamin B12;
- 28 mg iron; and
- a pharmaceutically acceptable carrier.

As outlined in more detail in the EXAMPLES section, below, particularly good results were surprisingly obtained with patients who were previously subjected to gastric sleeve surgery and to whom a unit dose is administered once daily, said unit dose comprising 28 mg iron and 100 µg vitamin B12. That is to say, a unit dose of the pharmaceutical composition for use according to the invention, comprising 28 mg iron and 100 µg vitamin B12, increases blood serum levels of said compounds to a large extent in gastric sleeve surgery patients, when compared to patients after gastric sleeve surgery and to whom a standard multivitamin preparation was administered.

It is further preferred to administer a unit dose of the composition according to the present invention once daily to a gastric sleeve patient, said unit dose preferably comprising 28 mg iron and 100 µg vitamin B12, according to the invention. These latter doses also increase blood serum levels of these vitamin B12 and iron to a level significant for health for patients after gastric sleeve surgery, though not exceeding health-threatening serum levels in said patients.

Said unit dose may further comprise between 150 µg and 1000 µg folic acid, preferably between 400 and 600 µg of folic acid.

Preferably, the pharmaceutical composition for use according to the invention is a composition wherein a unit dose of the composition further comprises:
- between 400 µg and 2000 µg of vitamin A;
- between 1 mg and 5,5 mg of vitamin B1;
- between 0,5 mg and 4 mg copper;
- between 7 mg and 60 mg zinc; and
- between 150 µg and 1000 µg folic acid, preferably between 400 and 600 µg.

The pharmaceutical composition for use according to the invention may also be a composition according to the present invention wherein a unit dose of the composition further comprises:
- between 1 mg and 5,5 mg of vitamin B1;
- between 7 mg and 60 mg zinc; and
- between 150 µg and 1000 µg folic acid, preferably between 400 and 600 µg.

Preferably, in the pharmaceutical composition for use according to the invention, the composition comprises between 400 µg and 2000 µg of vitamin A, more preferably between 800 µg and 1000 µg. Particularly preferred is a pharmaceutical composition for use according to the invention, wherein the composition comprises 800 µg of vitamin A or comprises 1000 µg of vitamin A.

Preferably, in the pharmaceutical composition for use according to the invention, the composition comprises between 0,5 mg copper and 4 mg copper in the form of copper gluconate, more preferably between 1 mg and 2 mg. Particularly preferred is a pharmaceutical composition for use according to the invention, wherein the composition comprises 1 mg copper or comprises 1,9 mg copper.

It has now been found in a study with patients who underwent gastric sleeve surgery that administering multivitamin preparations comprising about 2,75 mg vitamin B1 resulted in particularly stable and sufficient blood serum levels with regard to vitamin B1. In addition, when said patients were administered multivitamin preparations comprising about 500 µg vitamin B11 (i.e. folic acid), blood serum levels after about six months of daily intake of said multivitamin preparations were adequate and sufficient from a health perspective.

In one embodiment, the pharmaceutical composition for use according to the invention is a composition wherein a unit dose of the composition further comprises:
- between 1 mg and 4 mg of vitamin B2;
- between 12 mg and 50 mg niacin;
- between 4,5 mg and 18 mg of vitamin B5;
- between 1 mg and 4 mg of vitamin B6;
- between 75 µg and 300 µg biotin;
- between 50 mg and 200 mg of vitamin C;
- between 3,5 µg and 75 µg of vitamin D3;
- between 6 mg and 24 mg of vitamin E;
- between 20 µg and 80 µg chromium;
- between 75 µg and 300 µg iodine;
- between 1,5 mg and 6 mg manganese;
- between 25 µg and 100 µg molybdenum; and
- between 27 µg and 110 µg selenium.

Preferred is the pharmaceutical composition for use according to the invention, wherein the composition comprises about 75 microgram of vitamin D3. Also preferred is the pharmaceutical composition for use according to the invention, wherein the composition comprises between 1 mg and 3 mg vitamin B6, such as about 2 mg vitamin B6. A source for vitamin B6 in the composition for use of the invention is preferably pyridoxine HCl or pyridoxal 5 phosphate. In preferred embodiments of the invention, the pharmaceutical composition for use of the invention does not comprise any one or more of choline, vitamin K, and the pharmaceutical composition for use according to the invention is preferably devoid of choline and vitamin K. Typically, such a pharmaceutical composition for use of the invention then comprises about 75 microgram vitamin D3 and about 2 mg vitamin B6. It is preferred that such a pharmaceutical composition for use according to the invention comprises about 100 microgram vitamin B12 and about 28 mg iron. Also preferred is the pharmaceutical composition for use of the invention, comprising 100 microgram vitamin B12 and 28 mg iron, about 75 microgram vitamin D3, and which is devoid of any one or more of choline, vitamin K, and preferably said composition is devoid of all of choline, vitamin K.

The inventors found that administering to patients a multivitamin and multimineral composition comprising the amounts of minerals and vitamins according to the invention, as listed here above, surprisingly resulted in blood serum levels of iron and vitamin B12 that are acknowledged as being sufficient levels for sustained health, while the doses of all listed other vitamins and minerals did not result in hypovitaminosis nor in any hypervitaminosis. Thus, according to the invention, such pharmaceutical compositions of the invention are adequate and beneficial for patients who underwent gastric sleeve surgery and are therefore at risk for hypovitaminosis with regard to vitamin B12 and iron, and are at the same time at risk for hypervitaminosis with regard to e.g. vitamin A, vitamin B1 and vitamin B6 when they would take standard multivitamin preparations.

Moreover, it is now due to the inventors that a pharmaceutical composition for use according to the invention is made available that also aids in sustained blood serum levels of albumin in patients who underwent LSG, while at the same time haemoglobin (Hb) levels, hematocrit (Ht) levels, mean corpuscular volume (MCV) as well as parathyroid hormone (PTH) levels remain at values aimed for, for healthy subjects. That is to say, by administering the pharmaceutical composition for use according to the invention to LSG patients, risk for vitamin B12 deficiency and risk for iron deficiency is adequately conquered, whereas the risk for adverse side effects that could putatively be introduced by administering said composition to said patients, is absent.

It is also part of the invention that the pharmaceutical composition for use according to the invention optionally comprises for example about 10 mg choline.

In some embodiments, it is preferred that for the pharmaceutical composition for use according to the invention, a unit dose of the composition further comprises choline at an amount of 20 mg or less. Alternatively, the pharmaceutical composition for use according to the invention is devoid of choline.

The pharmaceutical composition for use in the treatment or prevention of vitamin deficiencies and mineral deficiencies in a patient who has been subjected to gastric sleeve surgery according to the invention is thus primarily suitable for administering to a patient who underwent said surgery. Without wishing to be bound by theory, for obese patients, including choline in the pharmaceutical composition of the invention can be beneficial pre-operative and post-operative, since it is suggested that choline may have liver-shrinking activity towards non-alcoholic fatty liver. Although, as said before, it is preferred that the pharmaceutical composition for use according to the invention is devoid of choline.

In an embodiment according to the present invention, the pharmaceutical composition is a composition that is administered at least once daily to patients in need thereof. Of course, it is also possible that a unit dose of the pharmaceutical composition according to the invention is administered twice, thrice, *etc.* daily, wherein such a unit dose comprises half, one third, *etc.*, of the daily dosage of the pharmaceutical composition.

In a further embodiment of the present invention, the pharmaceutical composition is formulated as a fixed dose combination, preferably as an oral fixed dose combination. Such oral fixed dose combination is provided by methods commonly known by those skilled in the art. Preferably, such oral fixed dose combination is provided as a solid dosage form, although such a combination can also be provided as a capsule filled with liquid, a gum, or the like, according to the invention.

Preferably, the oral fixed dose combination is a solid dosage form, such as a capsule, a tablet or a powder, which are relatively easy to administer. Preferably, a capsule is provided, according to methods known in the art, wherein such capsule is for example provided in a form facilitating easy swallowing, and/or provided with a lubricant for unhindered and fast traveling through the esophagus.

Particularly preferred is the pharmaceutical composition for use according to the invention, wherein a unit dose of the composition comprises about 75 microgram vitamin D3. Vitamin D3 is commonly administered to patients subjected to gastric sleeve surgery. That is to say, patients are administered for example twice daily a tablet comprising calcium and vitamin D accumulating to a daily dose of 500 mg calcium and 44 microgram (880 IE) vitamin D. In addition, the patients receiving twice per day a vitamin D tablet further receive a weekly supplement comprising 50.000 IU vitamin D, or 1.250 microgram vitamin D. It is part of the invention that the unit dose of the pharmaceutical composition comprises 75 microgram vitamin D3, such that patients who are subjected to gastric sleeve surgery do not have to take further vitamin D supplements. The amount of 75 microgram vitamin D in a unit dose is sufficient and enough for providing the patient who underwent the surgery with adequate plasma vitamin D levels similar to healthy untreated subjects.

A second aspect of the invention relates to a pharmaceutical composition comprising per unit dose:
- between 50 µg and 150 µg of vitamin B12;
- between 20 mg and 30 mg iron; and
- a pharmaceutically acceptable excipient, wherein the pharmaceutical composition is devoid of vitamin K and wherein the pharmaceutical composition is devoid of magnesium and wherein the composition is devoid of calcium, wherein a unit dose of said composition further comprises:
- between 400 µg and 2000 µg of vitamin A;
- between 1 mg and 5,5 mg of vitamin B1;
- between 0,5 mg and 4 mg copper;
- between 7 mg and 60 mg zinc; and
- between 150 µg and 1000 µg folic acid.

Said composition preferably comprises between 400 µg and 600 µg folic acid.

It is preferred that the pharmaceutical composition according to the invention is devoid of choline.

Without wishing to be bound by theory, regularly patients who has been subjected to gastric sleeve surgery are not at risk for developing a shortage of magnesium, i.e. too low magnesium levels in the circulation. First, magnesium is ubiquitously present in numerous food products regularly consumed by said patients. These food products comprise amounts of magnesium that commonly provide the patient who consumes said food products with sufficient amounts of magnesium. Second, without wishing to be bound by theory, after the gastric sleeve surgery, magnesium is still sufficiently and efficiently absorbed and taken up by the patient's body since the duodenum and the jejunum of the patient still function properly after surgery. Therefore, provision of magnesium to the patient after gastric sleeve surgery by administering a pharmaceutical composition comprising magnesium, is not a requirement for the maintenance of sufficient plasma levels of magnesium. The pharmaceutical composition of the invention is devoid of magnesium. In addition, magnesium provided as the salt magnesium citrate is in its dry form a relatively bulky and voluminous substance, that enlarges the volume of e.g. a capsule containing a unit dose of the pharmaceutical composition comprising the magnesium-citrate, considerably. Therefore, a capsule containing a unit dose of the pharmaceutical composition of the invention without magnesium is smaller and is administered to the patient more easily and more conveniently. This aspect contributes to improved patient compliance.

Particularly preferred is the pharmaceutical composition according to the invention, wherein a unit dose of the composition comprises:
- 100 µg of vitamin B12; and
- 28 mg iron.

As said, the inventors surprisingly found a pharmaceutical composition for administration to patients who underwent gastric sleeve surgery, such that vitamin B12 deficiency is prevented or reversed, and such that iron deficiency is prevented or reversed, while at the same time no hypervitaminosis with regard to other vitamins comprised by the pharmaceutical compositions of the invention, occurs, nor hypovitaminosis.

In addition, Table 1 displays two typical pharmaceutical compositions of the invention, said compositions being administered to patients who underwent LSG and were treated with the pharmaceutical composition of the invention for at least six month. During said time period of six month, vitamin B12 levels in blood serum and iron levels improved and reached levels generally accepted as not inflicting a health risk. Furthermore, in patients who underwent LSG, Hb levels, Ht levels, MCV as well as PTH levels remain at values aimed for, for healthy subjects, when the pharmaceutical composition of the invention is administered to said patients, for example for a time period of at least six months.

| TABLE 1. Exemplary pharmaceutical composition of the invention providing increased blood serum levels of at least vitamin B12 and iron in patients subjected to gastric sleeve surgery, such that vitamin B12 levels and iron levels reach the values for healthy subjects | | | |
|---|---|---|---|
| | | Exemplary multivitamin Composition 'GS-B' according to the invention | |
| **Active ingredient** | **Applied form of the Ingredient** | **Weight per capsule (unit dose)** | **RDA per capsule (unit dose)** |
| Vitamin A | Retiol palmitate | 800 microgram | 100% |
| Vitamin B 1 | Thiamine HCL | 2,75 mg | 250% |
| Vitamin B2 | Riboflavin | 2 mg | 143% |
| Niacin | Nicotinamide | 25 mg | 156% |
| Vitamin B5 | Calcium pantothenate | 9 mg | 150% |
| Vitamin B6 | Pyridoxine HCL | 2 mg | 143% |
| Biotin | Biotin | 150 microgram | 300% |
| Folic acid | Pteroylmonoglutamin acid | 500 microgram | 250% |
| Vitamin B12 | Cyanocobalamin | 100 microgram | 4000% |
| Vitamin C | Ascorbic acid | 100 mg | 125% |
| Vitamin D3 | Cholecalciferol | 7,5 microgram | 150% |
| Vitamin E | Tocopherol succinate | 12 mg | 100% |
| | | | |
| Chromium | Chromium III Chloride | 40 microgram | 100% |
| Iron | Iron fumerate | 28 mg | 200% |
| Iodine | Potassium iodide | 150 microgram | 100% |
| Copper | Copper gluconate | 1,9 mg | 190% |
| | | | |
| Manganese | Manganese citrate | 3 mg | 150% |
| Molybdenum | Sodium molybdate | 50 microgram | 100% |
| Selenium | Sodium selenite | 55 microgram | 100% |
| Zinc | Zinc citrate | 28 mg | 280% |

Preferred is a pharmaceutical composition for use of the invention, wherein the composition comprises between 1 mg and 3 mg vitamin B6, such as about 2 mg vitamin B6. A source for vitamin B6 in the composition for use of the invention is preferably pyridoxine HCl or pyridoxal 5 phosphate.

Also preferred is a pharmaceutical composition for use of the invention, wherein the composition comprises about 75 microgram vitamin D3.

In one embodiment, the pharmaceutical composition according to the invention is a composition, wherein a unit dose of the composition comprises:
- between 50 µg and 150 µg of vitamin B12, preferably 100 µg of vitamin B12; and
- between 20 mg and 30 mg iron, preferably 28 mg iron

In one embodiment, the pharmaceutical composition according to the invention, is a composition wherein a unit dose of said composition comprises:
- between 400 µg and 600 µg folic acid.

In one embodiment, the pharmaceutical composition according to the invention is a composition, wherein a unit dose of said composition further comprises:
- between 1 mg and 4 mg of vitamin B2;
- between 12 mg and 50 mg niacin;
- between 4,5 mg and 18 mg of vitamin B5;
- between 1 mg and 4 mg of vitamin B6;
- between 75 µg and 300 µg biotin;
- between 50 mg and 200 mg of vitamin C;
- between 3,5 µg and 75 µg of vitamin D3;
- between 6 mg and 24 mg of vitamin E;
- between 20 µg and 80 µg chromium;
- between 75 µg and 300 µg iodine;
- between 1,5 mg and 6 mg manganese;
- between 25 µg and 100 µg molybdenum; and
- between 27 µg and 110 µg selenium.

In one embodiment, the pharmaceutical composition according to the invention is a composition, wherein a unit dose of the composition further comprises:
- between 0 mg and 20 mg choline, preferably about 10 mg.

It is preferred that the pharmaceutical composition according to the invention is provided as a unit dose wherein a unit dose of the composition further comprises choline at an amount of 20 mg or less. The pharmaceutical composition of the invention is primarily suitable for administering to a patient who underwent gastric sleeve surgery. Without wishing to be bound by theory, for obese patients, including choline in the pharmaceutical composition of the invention can be beneficial pre-operative and post-operative, since it is suggested that choline may have liver-shrinking activity towards non-alcoholic fatty liver. Although, as said before, it is preferred that the pharmaceutical composition of the invention is devoid of choline.

Particularly preferred is the pharmaceutical composition according to the invention, wherein a unit dose of the composition comprises about 75 microgram vitamin D3. As said here above before, vitamin D3 is commonly administered to patients subjected to gastric sleeve surgery. That is to say, patients are administered for example twice daily a tablet comprising calcium and vitamin D accumulating to a daily dose of 500 mg calcium and 44 microgram (880 IE) vitamin D. In addition, the patients receiving twice per day a vitamin D tablet further receive a weekly supplement comprising 50.000 IU vitamin D, or 1.250 microgram vitamin D. It is therefore part of the invention that the unit dose of the pharmaceutical composition comprises 75 microgram vitamin D3, such that patients who are subjected to gastric sleeve surgery do not have to take further vitamin D supplements.

The amount of 75 microgram vitamin D in a unit dose is sufficient and enough for providing the patient who underwent the surgery with adequate plasma vitamin D levels similar to healthy untreated subjects. Including an amount of about 75 microgram vitamin D3 in e.g. a capsule containing a unit dose of the pharmaceutical composition of the invention, contributes to improved patient compliance since the separate administration of vitamin D to the patient, in addition to administration of the pharmaceutical composition of the invention, is not necessary anymore. Less compositions, i.e. tablets or capsules, etc., have to be administered to the patient, contributing to a more convenient dosing regimen and to less volume to be administered to the patient in order to be provided with the suitable dose of the minerals and vitamins.

To further lower the risk for hyper-vitaminosis of vitamin B6, a unit dose of the pharmaceutical composition for use of the invention preferably comprises less than 3 mg vitamin B6, and about 2 mg vitamin B6 per unit dose is typically applied.

In order to avoid interactions between vitamin B12, iron and calcium, calcium is absent in the pharmaceutical composition of the invention.

Vitamin K is a known coagulant and may have a negative effect on anti-coagulants provided to patients who underwent LSG after they have been operated. Furthermore, with respect to vitamin K, it is noted that a small amount may be needed for the body to be able to take up calcium. However, too much vitamin K may impair the function of anti-coagulants provided after the gastric sleeve procedure.

Although patients having been subjected to a gastric sleeve operation often suffer from a lack or deficiency of vitamin B12 and often suffer from iron deficiency, said patients are also at risk for developing other deficiencies with regard to minerals and vitamins. In order to prevent this, the pharmaceutical composition according to the present invention, and in particular a unit dose thereof, also comprises the above mentioned vitamins and minerals.

With respect to the use of zinc it is noted that additional zinc is preferred in order to avoid loss of hair after the surgery has been carried out.

Besides the above mentioned minerals and vitamins, the pharmaceutical composition according to the present invention also comprises a pharmaceutically acceptable excipient.

Such an excipient is chosen from ingredients which are commonly used in the pharmaceutical technology for preparing granulate, solid or liquid oral dosage formulations.

Examples of categories of excipients include, but are not limited to, binders, disintegrants, lubricants, glidants, fillers and diluents. One of ordinary skill in the art may select one or more of the aforementioned excipients with respect to the particular desired properties of the granulate e.g. from which capsules / tablets are produced, and/or solid oral dosage form by routine experimentation and without any undue burden. The amount of each excipient used may vary within ranges conventional in the art. The following references disclose techniques and excipients used to formulate oral dosage forms. See "The Handbook of Pharmaceutical Excipients", 4th edition, Rowe et al., Eds., American Pharmaceuticals Association (2003); and "Remington: The Science and Practice of Pharmacy", 20th edition, Gennaro, Ed., Lippincott Williams & Wilkins (2000).

The provision of extra vitamin D, calcium and optionally vitamin K may be of importance to patients which have been subjected to a bariatric surgery procedure because after the operation the pressure on weight-bearing bones is reduced by the rapid weight loss. This reduction causes lower stimulation of the osteoclasts, which in turn causes osteoblasts to produce less bone. This first phase lasts about three months. A second phase of bone resorption takes place six months postoperatively. This phase is due to a lack of intake of vitamin D and an insufficient absorption of calcium. The response of the body to the shortage of calcium is to absorb calcium from the skeleton reducing its strength. After one year, the bone loss in all the bones, especially in weight-bearing bones like the pelvis and lower back, has become significantly large at 7.8% to 10.5%. In the long term this is very detrimental to the patient as they develop a significantly greater risk of fractures. Hence, additional vitamin D may be needed for this group of patients.

The pharmaceutical composition according to the present invention is preferably administered at least once per day. In special cases, the combination may be administered more times a day, although it is assumed that this is only beneficial for a limited amount of time, *e.g.* one to three months.

A preferred embodiment is the pharmaceutical composition according to the invention wherein a unit dose of said composition comprises:

| **Active ingredient** | **Applied form of the Ingredient** | **Weight per capsule (unit dose)** |
|---|---|---|
| Vitamin A | Retiol palmitate | 800 microgram |
| Vitamin B 1 | Thiamine HCL | 2,75 mg |
| Vitamin B2 | Riboflavin | 2 mg |
| Niacin | Nicotinamide | 25 mg |
| Vitamin B5 | Calcium pantothenate | 9 mg |
| Vitamin B6 | Pyridoxine HCL | 2 mg |
| Biotin | Biotin | 150 microgram |
| Folic acid | Pteroylmonoglutamin acid | 500 microgram |
| Vitamin B12 | Cyanocobalamin | 100 microgram |
| Vitamin C | Ascorbic acid | 100 mg |
| Vitamin D3 | Cholecalciferol | 75 microgram |
| Vitamin E | Tocopherol succinate | 12 mg |
| | | |
| Chromium | Chromium III Chloride | 40 microgram |
| Iron | Iron fumerate | 28 mg |
| Iodine | Potassium iodide | 150 microgram |
| Copper | Copper gluconate | 1,9 mg |
| | | |
| Manganese | Manganese citrate | 3 mg |
| Molybdenum | Sodium molybdate | 50 microgram |
| Selenium | Sodium selenite | 55 microgram |
| Zinc | Zinc citrate | 28 mg |

It is thus preferred that the pharmaceutical composition of the invention is provided as a unit dose, such as a capsule, wherein the amount of vitamin D3 represents 1.500% of the RDA per unit dose (capsule).

Preferred is the pharmaceutical composition according to the invention, wherein the composition comprises about 75 microgram of vitamin D3. Also preferred is the pharmaceutical composition according to the invention, wherein the composition comprises between 1 mg and 3 mg vitamin B6, such as about 2 mg vitamin B6. A source for vitamin B6 in the composition of the invention is preferably pyridoxine HCl or pyridoxal 5 phosphate. In preferred embodiments of the invention, the pharmaceutical composition of the invention is devoid of choline. Typically, such a pharmaceutical composition of the invention then comprises about 75 microgram vitamin D3 and about 2 mg vitamin B6. It is preferred that such a pharmaceutical composition according to the invention comprises 100 microgram vitamin B12 and 28 mg iron. Also preferred is the pharmaceutical composition of the invention, comprising 100 microgram vitamin B12 and 28 mg iron, about 75 microgram vitamin D3, and which is devoid of choline.

A last aspect of the invention relates to the pharmaceutical composition of the invention for treating vitamin- and mineral deficiencies in patients who underwent gastric sleeve surgery.

The invention is further illustrated by the following examples, which should not be interpreted as limiting the present invention in any way.

### EXAMPLES

### Double blind, randomized study comparing two pharmaceutical compositions 'GS-B') according to the present invention with a standard multivitamin supplement ('R')

A double-blind, randomized, six-month study was conducted comparing a multivitamin/multimineral compositions 'GS-B' of the invention with a standard multivitamin supplement ('R') containing approximately 100% of recommended daily allowance (RDA) for iron, vitamin B12 and folic acid (See Table 1 and Table 2 for details). GS-B contains vitamin B12 at a level 4000% RDA, iron at 200% RDA, and folic acid at 250% RDA per capsule.

### Patients

A total of 98 patients who were scheduled for gastric sleeve surgery were randomized for postoperative multivitamin/multimineral supplementation for a duration of six months. All patients met the criteria for bariatric surgery according to NIH Consensus Development Conference Panel for bariatric surgery. Patients were randomized to receive either a standard multivitamin supplement (see Table 2 for the composition referred to as 'R') or gastric-sleeve surgical procedure specific multivitamin supplements (GS-B, see Table 1 and Table 2 for the compositions). A computer-generated variable block schedule was used for randomization. Patients, surgeons and researchers were blinded for the supplements. The supplements were similar for color, size, and packaging, and all three were dosed as one capsule daily. The composition R served as control and contained the compounds of interest in a dose about equivalent to the recommended daily allowance (RDA), whereas GS-B contained increased doses for several of the minerals and vitamins, in particular, of iron (1,5 or 2,0 times RDA) and vitamin B12 (4 or 40 times RDA).

### Surgical procedure

All procedures were performed by one of three bariatric surgeons. They performed a gastric sleeve surgical procedure. All patients received low-molecular heparin (Nadroparin 5700IU daily) for six weeks and proton-pump inhibitor (Omeprazol 40 mg daily) for six months, as a part of a standard postoperative protocol.

### Follow-up and outcome

All patients followed a strict postoperative schedule consisting of several visits in the first six months, and on each visit patients were encouraged to keep taking their supplements. Standard laboratory blood test were performed at baseline, and at 6 months. This included a complete blood count (CBC, (normal range (NR) haemoglobin: females: 7.4 - 9.9 mmol/L, males: 8.4 - 10.8 mmol/L), mean cell volume (MCV, [NR: 80 - 100 fL]), creatinine [NR: 45 - 90 µmol/L], sodium [NR: 135 - 145 mmol/l], potassium [NR: 3.5 - 4.7 mmol/l], calcium [NR: 2.10 - 2.55 mmol/l], phosphate [NR: 0.87 - 1.45 mmol/l], magnesium [NR: 0.71 - 0.93 mmol/l], zinc [NR 9.2 - 18.4 µmol/L], albumin [35 - 50 g/l], fasting glucose (FG, [NR: 4.0 - 5.6 mmol/l]), HblAc [NR: 20 - 42 mmol/mol], total cholesterol [NR <6.50 mmol/l] , HDL-cholesterol [NR >1.10 mmol/l], LDL-cholesterol [NR: 3.50 - 4.50 mmol/l], and triglycerides [NR: 0.8 - 2.0 mmol/l]), iron [NR: 9.0 - 31.0 µmol/l], total-iron-binding-capacity (TIBC, [NR 45.0 - 81.0 µmol/L]), ferritin [NR 20 -200 µg/L], folic acid [NR: 9.0 - 36.0 nmol/l], vitamin B12 [NR: 150 - 640 pmol/l], 25-hydroxyvitamin D (25-OHD, [NR: >50 nmol/L]), parathyroid hormone (PTH, [NR: 1.3 - 6.8 pmol/l]), vitamin B1 [NR: 95 - 175 nmol/L], vitamin B6 [NR 25 - 100 nmol/L].

Primary outcome variables were the percentage of iron and vitamin B12 deficiencies developed during the six month after gastric sleeve surgery. Iron Deficiency (ID), was defined as a serum ferritin < 20 µg/L and vitamin B12 deficiency if the level was <150 pmol/L). Vitamin D deficiency was diagnosed if 25-OHD < 50 nmol/L, hypocalcemia if serum calcium <2.1 mmol/L), and zinc deficiency of the serum level was <9.2 µmol/L. Calcium data are shown as calcium levels corrected for albumin 130 (Cacorr), according to the following equation: Ca_{corr} = Total Calcium - (0.025 x albumin) + 1.

### Statistical analysis

Sample size calculation was performed by the epidemiologist of the Research Department of Rijnstate Hospital in Arnhem (NL) using Openepi.com. Sample size calculation was based on the number of patients developing ID.

All data were analyzed using IBM^{®} SPSS^{®} Statistics 22.0 for Windows. Data were expressed in mean (± standard deviation), unless otherwise specified. Difference between groups were calculated using student-t test for continuous data and chi-square test for ordinal/ nominal data. A P-value < 0.05 was considered statistically significant.

Tables 3-5 show the initial blood serum values for the listed vitamins, minerals, proteins, determined at the start of the testing of the effects of the pharmaceutical composition SG-B on patients who underwent gastric sleeve surgery.

In Tables 6 and 7, measured blood serum levels for the listed vitamins, minerals, proteins are provided, as determined at time point six months after the start of the clinical trial, said trial comprising administering either standard multivitamin composition R to patients who underwent LSG, or either of multivitamin/multimineral composition of the invention, *i.e.* GS-B.

### Results

In total 98 patients 70 in the 'R'control group; 28 in the GS-B group) underwent a gastric sleeve surgical procedure and were included for analysis. The two groups were similar with respect to age, sex, weight, body mass index (BMI) and preoperative deficiencies.

### Weight loss

The degree of weight loss over six months was similar in the two groups. Weight dropped from 142.2 (±31.8) to 108.7 (±27.0) in the 'R' control group and from 139.8 (±26.8) to 104.1 (±23.3) in the GS-B group. Percentage Excess Weight Loss (%EWL), defined as weight loss divided by excess weight based on ideal body weight at BMI 25 kg/m², were after six months was 52.4% (±19.7) and 57.1 (±16.6) for 'R' control group and GS-B group, respectively.

### Iron

The total number of patients developing ferritin deficiency during follow-up was 1 patient in the R group and no in the GS-B group. Mean serum ferritin levels increased slightly (difference (delta) between start and after 6 months was 5.8 µg/L) in patients in the 'R'control group, whereas the ferritin levels increased considerably in the patients of the GS-B group (difference (delta) between start and after 6 months was 56.8 µg/L).

### Vitamin B12

In the 'R' control group five patients developed vitamin B12 deficiencies during the trial. In the GS-B group none of the patients developed a B12 deficiency. Furthermore the mean vitamin B12 levels in the patients of the 'R' control group decreased from 317.3 (±110.7) pmol/L to 292.3 (±93.4) pmol/L, whereas the B12 levels in the GS-B group increased from 312.4 (±228.5) pmol/L to 343.9 (±97.9) pmol/L. Clearly, the patients in the 'R'control group were at a considerable risk to develop B12 deficiencies, and in fact some of them already did. This did not occur in the GS-B group.

| TABLE 2. Multivitamin and multimineral compositions included in clinical tests with patients previously subjected to gastric sleeve surgery. | | | | |
|---|---|---|---|---|
| | reference multivitamin capsule 'R' (unit dose) recommended for daily use by healthy subjects | | Exemplary multivitamin Composition 'GS-B' according to the invention | |
| | | | | |
| **Active ingredient^{‡}** | **Weight per capsule (unit dose)^{†}** | **RDA per capsule (unit dose)^{†}** | **Weight per capsule (unit dose)** | **RDA per capsule (unit dose)** |
| Vitamin A | 600 µg | 75% | 800 µg | 100% |
| Vitamin B1 | 1,1 mg | 100% | 2,75 mg | 250% |
| Vitamin B2 | 1,4 mg | 100% | 2 mg | 143% |
| Niacin | 16 mg | 100% | 25 mg | 156% |
| Vitamin B5 | 6 mg | 100% | 9 mg | 150% |
| Vitamin B6 | 1,4 mg | 100% | 2 mg | 143% |
| Biotin | 25 µg | 50% | 150 µg | 300% |
| Folic acid | 200 µg | 100% | 500 µg | 250% |
| Vitamin B12 | 2,5 µg | 100% | 100 µg | 4000% |
| Vitamin C | 80 mg | 100% | 100 mg | 125% |
| Vitamin D3 | 4 µg | 80% | 7,5 µg | 150% |
| Vitamin E | 10 mg | 83% | 12 mg | 100% |
| Vitamine K1 | 25 µg | 33% | x | x |
| Chromium | 40 µg | 100% | 40 µg | 100% |
| Iron (Fe) | 14 mg | 100% | 28 mg | 200% |
| Iodine (I) | 154 µg | 103% | 150 µg | 100% |
| Copper (Cu) | 1 mg | 100% | 1,9 mg | 190% |
| Magnesium (Mg) | 16 mg | 4% | x | x |
| Manganese | 2 mg | 100% | 3 mg | 150% |
| Molybdenum | 50 µg | 100% | 50 µg | 100% |
| Selenium (Se) | 55 µg | 100% | 55 µg | 100% |
| Zinc (Zn) | 10 mg | 100% | 28 mg | 280% |
| Calcium (Ca) | 40 mg | 5% | x | x |
| Q10 | 10 mg | x | x | x |
| ‡ See Table 1 for the applied form of the active ingredients | | | | |
| † 'x' refers to the absence of the indicated active ingredient in a composition d | | | | |

| TABLE 3. Blood levels for vitamins, minerals before treatment of gastric sleeve patients with the reference multivitamin capsule 'R', comprising about 100% of the RDA of vitamins and minerals (See Table 2) | | | | | |
|---|---|---|---|---|---|
| Descriptive Statistics | | | | | |
| | N | Minimum | Maximum | Mean | Std. Deviation |
| Haemoglobin (Hb) (mM/L) | 40 | 6.1 | 10.3 | 8.743 | .8409 |
| Hematocrit (Ht) | 40 | .32 | .49 | .4273 | .03559 |
| Mean corpuscular volume (MCV) | 39 | 58.0 | 101.0 | 88.282 | 7.0560 |
| phosphate | 36 | .65 | 1.39 | .9450 | .17785 |
| Ca | 37 | 2.09 | 2.45 | 2.2884 | .08700 |
| Mg | 36 | .66 | .89 | .8022 | .04794 |
| Albumin | 37 | 31.0 | 42.0 | 37.405 | 2.6922 |
| Zinc | 34 | 7.2 | 14.4 | 11.418 | 1.5797 |
| Parathyroid hormone (PTH) | 40 | .8 | 11.2 | 3.818 | 2.3881 |
| Vitamin D | 40 | 16.0 | 87.0 | 37.575 | 15.6039 |
| Vitamin B11 | 40 | 6.5 | 41.0 | 16.580 | 6.0882 |
| Vitamin B12 (pmol/L) | 37 | 200.0 | 538.0 | 334.919 | 103.1494 |
| Iron (Fe) | 38 | 21.0 | 406.0 | 118.661 | 89.5438 |
| Vitamin B 1 | 36 | 95.0 | 240.0 | 159.417 | 33.2406 |
| Vitamin B6 | 36 | 43.0 | 200.0 | 75.250 | 31.8383 |
| Vitamin A | 0 | | | | |
| Valid N (listwise) | 0 | | | | |

| TABLE 4. Blood levels for vitamins, minerals before treatment of gastric sleeve patients with the exemplary multivitamin Composition GS-B according to the invention, comprising adjusted levels of minerals and vitamins compared to the reference multivitamin capsule 'R' (See Table 2) | | | | | |
|---|---|---|---|---|---|
| Descriptive Statistics | | | | | |
| | N | Minimum | Maximum | Mean | Std. Deviation |
| Haemoglobin (Hb) | 26 | 7.6 | 10.3 | 8.973 | .7286 |
| Hematocrit (Ht) | 26 | .38 | .50 | .4477 | .03362 |
| Mean corpuscular volume (MCV) | 26 | 78.0 | 97.0 | 88.962 | 4.3770 |
| phosphate | 20 | .70 | 73.00 | 4.4725 | 16.12996 |
| Ca | 23 | 2.17 | 2.48 | 2.3183 | .07744 |
| Mg | 20 | .64 | .88 | .7985 | .05441 |
| Albumin | 23 | 31.0 | 43.0 | 38.000 | 3.0000 |
| Zinc | 25 | 5.4 | 81.0 | 13.476 | 14.2658 |
| Parathyroid hormone (PTH) | 26 | 1.4 | 6.1 | 3.158 | 1.2455 |
| Vitamin D | 26 | 10.0 | 101.0 | 42.692 | 24.4913 |
| Vitamin B11 | 26 | 9.4 | 35.9 | 16.762 | 6.3392 |
| Vitamin B12 | 21 | 202.0 | 407.0 | 307.524 | 61.3381 |
| Iron (Fe) | 26 | 22.0 | 415.0 | 137.885 | 113.2496 |
| Vitamin B 1 | 25 | 137.0 | 234.0 | 170.520 | 26.7319 |
| Vitamin B6 | 25 | 47.0 | 96.0 | 72.200 | 12.8906 |
| Vitamin A | 15 | 1.09 | 3.68 | 1.7807 | .68037 |
| Valid N (listwise) | 13 | | | | |

| TABLE 5. Differences in blood levels for vitamins, minerals after treatment of gastric sleeve patients for six months with, either the exemplary multivitamin Composition GS-A according to the invention, or the exemplary multivitamin Composition GS-B according to the invention | | | | | |
|---|---|---|---|---|---|
| Group Statistics | | | | | |
| | Group † | N | Mean | Std. Deviation | Std. Error Mean |
| DeltaHb | GS-B | 24 | -.346 | .4672 | .0954 |
| Ht | GS-B | 23 | -.018 | .0229 | .0048 |
| MCV | GS-B | 23 | 1.000 | 2.8123 | .5864 |
| phosphate | GS-B | 11 | .182 | .1722 | .0519 |
| Ca | GS-B | 23 | .061 | .0871 | .0182 |
| Mg | GS-B | 11 | .044 | .0450 | .0136 |
| Albumin | GS-B | 23 | .696 | 2.4760 | .5163 |
| Zinc | GS-B | 15 | -1.980 | 18.8063 | 4.8558 |
| PTH | GS-B | 22 | .100 | 1.4648 | .3123 |
| Vitamin D | GS-B | 22 | 43.727 | 26.6783 | 5.6878 |
| Vitamin B11 | GS-B | 22 | 3.314 | 9.6242 | 2.0519 |
| | | | | | |
| Vitamin B12 | GS-B | 17 | 35.176 | 92.4866 | 22.4313 |
| | | | | | |
| Fe | GS-B | 22 | 34.591 | 84.1844 | 17.9482 |
| Vitamin B 1 | GS-B | 15 | -18.800 | 35.7775 | 9.2377 |
| Vitamin B6 | GS-B | 15 | 13.933 | 32.0033 | 8.2632 |
| | GS-B | 15 | 13.933 | 32.0033 | 8.2632 |
| | | | | | |

| TABLE 6. Differences in blood levels for vitamins, minerals after treatment of gastric sleeve patients for six months with, either the reference multivitamin capsule 'R', or the exemplary multivitamin Composition GS-B according to the invention | | | | | |
|---|---|---|---|---|---|
| **Group Statistics** | | | | | |
| | Groep | N | Mean | Std. Deviation | Std. Error Mean |
| DeltaHb | R | 40 | -.260 | .5592 | .0884 |
| | GS-B | 24 | -.346 | .4672 | .0954 |
| Ht | R | 40 | -.008 | .0286 | .0045 |
| | GS-B | 23 | -.018 | .0229 | .0048 |
| MCV | R | 39 | 1.949 | 2.5541 | .4090 |
| | GS-B | 23 | 1.000 | 2.8123 | .5864 |
| phosphate | R | 29 | .017 | .1727 | .0321 |
| | GS-B | 11 | .182 | .1722 | .0519 |
| Ca | R | 37 | .060 | .1075 | .0177 |
| | GS-B | 23 | .061 | .0871 | .0182 |
| Mg | R | 27 | .020 | .0696 | .0134 |
| | GS-B | 11 | .044 | .0450 | .0136 |
| Albumin | R | 37 | .811 | 3.9919 | .6563 |
| | GS-B | 23 | .696 | 2.4760 | .5163 |
| Zink | R | 24 | .204 | 2.5463 | .5198 |
| | GS-B | 15 | -1.980 | 18.8063 | 4.8558 |
| PTH | R | 39 | -.149 | 2.5837 | .4137 |
| | GS-B | 22 | .100 | 1.4648 | .3123 |
| Vitamin D | R | 39 | 61.026 | 43.9279 | 7.0341 |
| | GS-B | 22 | 43.727 | 26.6783 | 5.6878 |
| Vitamin B11 | R | 37 | 3.319 | 7.1877 | 1.1816 |
| | GS-B | 22 | 3.314 | 9.6242 | 2.0519 |
| Vitamin B12 | R | 31 | -38.226 | 73.6097 | 13.2207 |
| | GS-B | 17 | 35.176 | 92.4866 | 22.4313 |
| Fe | R | 31 | -5.484 | 58.1532 | 10.4446 |
| | GS-B | 22 | 34.591 | 84.1844 | 17.9482 |
| Vitamin B1 | R | 26 | -25.346 | 32.9921 | 6.4703 |
| | GS-B | 15 | -18.800 | 35.7775 | 9.2377 |
| Vitamin B6 | R | 25 | 12.120 | 28.7899 | 5.7580 |
| | GS-B | 15 | 13.933 | 32.0033 | 8.2632 |
| † 'GS-B' refers to the patient group that was subjected to gastric sleeve surgery and was subsequently administered a pharmaceutical composition 'GS-B' according to the invention for six months; 'R' refers to the patient group that was subjected to gastric sleeve surgery and was subsequently administered for six months a standard multimineral and multivitamin composition 'R' comprising vitamins and minerals at about 100% of the RDA (See Table 2). | | | | | |

## Claims

1. A pharmaceutical composition for use in the treatment or prevention of vitamin deficiencies and mineral deficiencies in a patient who has been subjected to gastric sleeve surgery comprising:
- vitamin B12 or a source thereof;
- iron or a source thereof;
- a pharmaceutically acceptable carrier; and
wherein the patient in need thereof is administered by means of said composition per day between 50 µg and 150 µg of vitamin B12, between 20 mg and 30 mg iron and less than 0,1 µg of vitamin K, and
wherein a unit dose of the pharmaceutical composition comprises:
- between 50 µg and 150 µg of vitamin B12;
- between 20 mg and 30 mg iron; and
- a pharmaceutically acceptable carrier;
wherein the pharmaceutical composition is devoid of calcium and is devoid of magnesium.

2. The pharmaceutical composition for use according to claim 1, wherein the pharmaceutical composition is devoid of vitamin K.

3. The pharmaceutical composition for use according to claim 1 or 2, wherein a unit dose of the composition comprises:
- 100 µg of vitamin B12; and
- 28 mg iron.

4. The pharmaceutical composition for use according to any of the claims 1 to 3, wherein a unit dose of the composition comprises about 2 mg vitamin B6.

5. The pharmaceutical composition for use according to any of the claims 1 to 4, wherein a unit dose of the composition comprises about 75 microgram vitamin D3.

6. The pharmaceutical composition for use according to any of the claims 1 to 5, wherein a unit dose of the composition further comprises:
- between 400 µg and 2000 µg of vitamin A;
- between 1 mg and 5,5 mg of vitamin B1;
- between 0,5 mg and 4 mg copper;
- between 20 mg and 60 mg zinc; and
- between 150 µg and 1000 µg folic acid.

7. The pharmaceutical composition for use according to any of the claim 1 to 3 or 6, wherein a unit dose of the composition further comprises:
- between 1 mg and 4 mg of vitamin B2;
- between 12 mg and 50 mg niacin;
- between 4,5 mg and 18 mg of vitamin B5;
- between 1 mg and 4 mg of vitamin B6;
- between 75 µg and 300 µg biotin;
- between 50 mg and 200 mg of vitamin C;
- between 3,5 µg and 75 µg of vitamin D3;
- between 6 mg and 24 mg of vitamin E;
- between 20 µg and 80 µg chromium;
- between 75 µg and 300 µg iodine;
- between 1,5 mg and 6 mg manganese;
- between 25 µg and 100 µg molybdenum; and
- between 27 µg and 110 µg selenium.

8. The pharmaceutical composition for use according to any of the claims 1 to 7, wherein a unit dose of the composition further comprises choline at an amount of 20 mg or less.

9. The pharmaceutical composition for use according to any of the claims 1 to 7, wherein the pharmaceutical composition is devoid of choline.

10. The pharmaceutical composition for use according to any of the previous claims, wherein the composition is administered at least once daily to patients in need thereof.

11. The pharmaceutical composition for use according to any of the previous claims, wherein the composition is formulated as a fixed dose combination, preferably as an oral fixed dose combination.

12. The pharmaceutical composition for use according to the previous claim, wherein the oral fixed dose combination is a solid dosage form, such as a capsule, a tablet or a powder.

13. A pharmaceutical composition comprising per unit dose:
- between 50 µg and 150 µg of vitamin B12;
- between 20 mg and 30 mg iron; and
- a pharmaceutically acceptable excipient, wherein the pharmaceutical composition is devoid of vitamin K and wherein the pharmaceutical composition is devoid of magnesium and wherein the composition is devoid of calcium,
wherein a unit dose of said composition further comprises:
- between 400 µg and 2000 µg of vitamin A;
- between 1 mg and 5,5 mg of vitamin B1;
- between 0,5 mg and 4 mg copper;
- between 7 mg and 60 mg zinc; and
- between 150 µg and 1000 µg folic acid.

14. The pharmaceutical composition according to claim 13, wherein the pharmaceutical composition is devoid of choline.

15. The pharmaceutical composition according to claim 13 or 14, wherein a unit dose of the composition comprises:
- 100 µg of vitamin B12; and
- 28 mg iron.

16. The pharmaceutical composition according to any of the claims 13-15, wherein a unit dose of said composition further comprises:
- between 1 mg and 4 mg of vitamin B2;
- between 12 mg and 50 mg niacin;
- between 4,5 mg and 18 mg of vitamin B5;
- between 1 mg and 4 mg of vitamin B6;
- between 75 µg and 300 µg biotin;
- between 50 mg and 200 mg of vitamin C;
- between 3,5 µg and 75 µg of vitamin D3;
- between 6 mg and 24 mg of vitamin E;
- between 20 µg and 80 µg chromium;
- between 75 µg and 300 µg iodine;
- between 1,5 mg and 6 mg manganese;
- between 25 µg and 100 µg molybdenum; and
- between 27 µg and 110 µg selenium.

17. The pharmaceutical composition according to any of the claims 13, 15 or 16, wherein a unit dose of the composition further comprises choline at an amount of 20 mg or less.

18. The pharmaceutical composition according to any of the claims 13-17, wherein a unit dose of the composition comprises about 75 microgram vitamin D3.

19. The pharmaceutical composition according to any of the claims 13-18, wherein a unit dose of the composition comprises about 2 mg vitamin B6.

## Patentansprüche

1. Pharmazeutische Zusammensetzung zur Verwendung bei der Behandlung oder Vorbeugung von Vitaminmangel und Mineralstoffmangel in einem Patienten, der sich einer Schlauchmagenoperation unterzogen hat, umfassend:
- Vitamin B12 oder eine Quelle davon;
- Eisen oder eine Quelle davon;
- einen pharmazeutisch akzeptablen Träger; und
wobei dem Patienten, der dies benötigt, mittels der Zusammensetzung täglich zwischen 50 µg und 150 µg Vitamin B12, zwischen 20 mg und 30 mg Eisen und weniger als 0,1 µg Vitamin K verabreicht wird, und
wobei eine Einheitsdosis der pharmazeutischen Zusammensetzung folgendes umfasst:
- zwischen 50 µg und 150 µg Vitamin B12,
- zwischen 20 mg und 30 mg Eisen, und
- einen pharmazeutisch akzeptablen Träger;
wobei die pharmazeutische Zusammensetzung frei von Calcium und frei von Magnesium ist.

2. Pharmazeutische Zusammensetzung zur Verwendung nach Anspruch 1, wobei die pharmazeutische Zusammensetzung frei von Vitamin K ist.

3. Pharmazeutische Zusammensetzung zur Verwendung nach Anspruch 1 oder 2, wobei eine Einheitsdosis der Zusammensetzung folgendes umfasst:
- 100 µg Vitamin B12; und
- 28 mg Eisen.

4. Pharmazeutische Zusammensetzung zur Verwendung nach einem der Ansprüche 1 bis 3, wobei eine Einheitsdosis der Zusammensetzung etwa 2 mg Vitamin B6 umfasst.

5. Pharmazeutische Zusammensetzung zur Verwendung nach einem der Ansprüche 1 bis 4, wobei eine Einheitsdosis der Zusammensetzung etwa 75 µg Vitamin D3 umfasst.

6. Pharmazeutische Zusammensetzung zur Verwendung nach einem der Ansprüche 1 bis 5, wobei eine Einheitsdosis der Zusammensetzung ferner folgendes umfasst:
- zwischen 400 µg und 2000 µg Vitamin A;
- zwischen 1 mg und 5,5 mg Vitamin B1;
- zwischen 0,5 mg und 4 mg Kupfer;
- zwischen 20 mg und 60 mg Zink; und
- zwischen 150 µg und 1000 µg Folsäure.

7. Pharmazeutische Zusammensetzung zur Verwendung nach einem der Ansprüche 1 bis 3 oder 6, wobei eine Einheitsdosis der Zusammensetzung ferner folgendes umfasst:
- zwischen 1 mg und 4 mg Vitamin B2;
- zwischen 12 mg und 50 mg Niacin;
- zwischen 4,5 mg und 18 mg Vitamin B5;
- zwischen 1 mg und 4 mg Vitamin B6;
- zwischen 75 µg und 300 µg Biotin;
- zwischen 50 mg und 200 mg Vitamin C;
- zwischen 3,5 µg und 75 µg Vitamin D3;
- zwischen 6 mg und 24 mg Vitamin E;
- zwischen 20 µg und 80 µg Chrom;
- zwischen 75 µg und 300 µg Jod;
- zwischen 1,5 mg und 6 mg Mangan;
- zwischen 25 µg und 100 µg Molybdän; und
- zwischen 27 µg und 110 µg Selen.

8. Pharmazeutische Zusammensetzung zur Verwendung nach einem der Ansprüche 1 bis 7, wobei eine Einheitsdosis der Zusammensetzung ferner Cholin in einer Menge von 20 mg oder weniger umfasst.

9. Pharmazeutische Zusammensetzung zur Verwendung nach einem der Ansprüche 1 bis 7, wobei die pharmazeutische Zusammensetzung frei von Cholin ist.

10. Pharmazeutische Zusammensetzung zur Verwendung nach einem der vorhergehenden Ansprüche, wobei die Zusammensetzung mindestens einmal täglich an Patienten, welche dies benötigen, verabreicht wird.

11. Pharmazeutische Zusammensetzung zur Verwendung nach einem der vorhergehenden Ansprüche, wobei die Zusammensetzung als Fixdosiskombination, vorzugsweise als orale Fixdosiskombination, formuliert ist.

12. Pharmazeutische Zusammensetzung zur Verwendung nach dem vorhergehenden Anspruch, wobei die orale Fixdosiskombination eine feste Darreichungsform ist, wie beispielsweise eine Kapsel, eine Tablette oder ein Pulver.

13. Pharmazeutische Zusammensetzung, die pro Einheitsdosis folgendes umfasst:
- zwischen 50 µg und 150 µg Vitamin B12;
- zwischen 20 mg und 30 mg Eisen; und
- einen pharmazeutisch annehmbaren Hilfsstoff,
wobei die pharmazeutische Zusammensetzung kein Vitamin K umfasst und wobei die pharmazeutische Zusammensetzung kein Magnesium umfasst und wobei die Zusammensetzung kein Calcium umfasst,
wobei eine Einheitsdosis der Zusammensetzung ferner folgendes umfasst:
- zwischen 400 µg und 2000 µg Vitamin A;
- zwischen 1 mg und 5,5 mg Vitamin B1;
- zwischen 0,5 mg und 4 mg Kupfer;
- zwischen 7 mg und 60 mg Zink; und
- zwischen 150 µg und 1000 µg Folsäure.

14. Pharmazeutische Zusammensetzung nach Anspruch 13, wobei die pharmazeutische Zusammensetzung frei von Cholin ist.

15. Pharmazeutische Zusammensetzung nach Anspruch 13 oder 14, wobei eine Einheitsdosis der Zusammensetzung folgendes umfasst:
- 100 µg Vitamin B12; und
- 28 mg Eisen.

16. Pharmazeutische Zusammensetzung nach einem der Ansprüche 13 bis 15, wobei eine Einheitsdosis der Zusammensetzung ferner folgendes umfasst:
- zwischen 1 mg und 4 mg Vitamin B2;
- zwischen 12 mg und 50 mg Niacin;
- zwischen 4,5 mg und 18 mg Vitamin B5;
- zwischen 1 mg und 4 mg Vitamin B6;
- zwischen 75 µg und 300 µg Biotin;
- zwischen 50 mg und 200 mg Vitamin C;
- zwischen 3,5 µg und 75 µg Vitamin D3;
- zwischen 6 mg und 24 mg Vitamin E;
- zwischen 20 µg und 80 µg Chrom;
- zwischen 75 µg und 300 µg Jod;
- zwischen 1,5 mg und 6 mg Mangan;
- zwischen 25 µg und 100 µg Molybdän; und
- zwischen 27 µg und 110 µg Selen.

17. Pharmazeutische Zusammensetzung nach einem der Ansprüche 13, 15 oder 16, wobei eine Einheitsdosis der Zusammensetzung ferner Cholin in einer Menge von 20 mg oder weniger umfasst.

18. Pharmazeutische Zusammensetzung nach einem der Ansprüche 13-17, wobei eine Einheitsdosis der Zusammensetzung etwa 75 µg Vitamin D3 umfasst.

19. Pharmazeutische Zusammensetzung nach einem der Ansprüche 13-18, wobei eine Einheitsdosis der Zusammensetzung etwa 2 mg Vitamin B6 umfasst.

## Revendications

1. Composition pharmaceutique destinée à être utilisée dans le traitement ou la prévention de carences en vitamines et de carences en minéraux chez un patient ayant été soumis à une gastrectomie comprenant :
- de la vitamine B12 ou une source de celle-ci ;
- du fer ou une source de celui-ci ;
- un vecteur pharmaceutiquement acceptable ; et
dans laquelle le patient qui en a besoin reçoit une administration au moyen de ladite composition, par jour, entre 50 µg et 150 µg de vitamine B12, entre 20 mg et 30 mg de fer et moins de 0,1 µg de vitamine K, et
dans laquelle une dose unitaire de la composition pharmaceutique comprend :
- entre 50 µg et 150 µg de vitamine B12 ;
- entre 20 mg et 30 mg de fer ; et
- un vecteur pharmaceutiquement acceptable ;
dans laquelle la composition pharmaceutique est exempte de calcium et est exempte de magnésium.

2. Composition pharmaceutique destinée à être utilisée selon la revendication 1, dans laquelle la composition pharmaceutique est exempte de vitamine K.

3. Composition pharmaceutique destinée à être utilisée selon la revendication 1 ou 2, dans laquelle une dose unitaire de la composition comprend :
- 100 µg de vitamine B12 ; et
- 28 mg de fer.

4. Composition pharmaceutique destinée à être utilisée selon l'une quelconque des revendications 1 à 3, dans laquelle une dose unitaire de la composition comprend environ 2 mg de vitamine B6.

5. Composition pharmaceutique destinée à être utilisée selon l'une quelconque des revendications 1 à 4, dans laquelle une dose unitaire de la composition comprend environ 75 microgrammes de vitamine D3.

6. Composition pharmaceutique destinée à être utilisée selon l'une quelconque des revendications 1 à 5, dans laquelle une dose unitaire de la composition comprend en outre :
- entre 400 µg et 2000 µg de vitamine A ;
- entre 1 mg et 5,5 mg de vitamine B1 ;
- entre 0,5 mg et 4 mg de cuivre ;
- entre 20 mg et 60 mg de zinc ; et
- entre 150 µg et 1000 µg d'acide folique.

7. Composition pharmaceutique destinée à être utilisée selon l'une quelconque de la revendication 1 à 3 ou 6, dans laquelle une dose unitaire de la composition comprend en outre :
- entre 1 mg et 4 mg de vitamine B2 ;
- entre 12 mg et 50 mg de niacine ;
- entre 4,5 mg et 18 mg de vitamine B5 ;
- entre 1 mg et 4 mg de vitamine B6 ;
- entre 75 µg et 300 µg de biotine ;
- entre 50 mg et 200 mg de vitamine C ;
- entre 3,5 µg et 75 µg de vitamine D3 ;
- entre 6 mg et 24 mg de vitamine E ;
- entre 20 µg et 80 µg de chrome ;
- entre 75 µg et 300 µg d'iode ;
- entre 1,5 mg et 6 mg de manganèse ;
- entre 25 µg et 100 µg de molybdène ; et
- entre 27 µg et 110 µg de sélénium.

8. Composition pharmaceutique destinée à être utilisée selon l'une quelconque des revendications 1 à 7, dans laquelle une dose unitaire de la composition comprend en outre de la choline en une quantité de 20 mg ou moins.

9. Composition pharmaceutique destinée à être utilisée selon l'une quelconque des revendications 1 à 7, dans laquelle la composition pharmaceutique est exempte de choline.

10. Composition pharmaceutique destinée à être utilisée selon l'une quelconque des revendications précédentes, dans laquelle la composition est administrée au moins une fois par jour à des patients qui en ont besoin.

11. Composition pharmaceutique destinée à être utilisée selon l'une quelconque des revendications précédentes, dans laquelle la composition est formulée sous la forme d'une combinaison de doses fixes, de préférence sous la forme d'une combinaison de doses fixes orales.

12. Composition pharmaceutique destinée à être utilisée selon la revendication précédente, dans laquelle la combinaison de doses fixes orales est une forme posologique solide, telle qu'une capsule, un comprimé ou une poudre.

13. Composition pharmaceutique comprenant par dose unitaire :
- entre 50 µg et 150 µg de vitamine B12 ;
- entre 20 mg et 30 mg de fer ; et
- un excipient pharmaceutiquement acceptable, dans laquelle la composition pharmaceutique est exempte de vitamine K et dans laquelle la composition pharmaceutique est exempte de magnésium et dans laquelle la composition est exempte de calcium,
dans laquelle une dose unitaire de ladite composition comprend en outre :
- entre 400 µg et 2000 µg de vitamine A ;
- entre 1 mg et 5,5 mg de vitamine B1 ;
- entre 0,5 mg et 4 mg de cuivre ;
- entre 7 mg et 60 mg de zinc ; et
- entre 150 µg et 1000 µg d'acide folique.

14. Composition pharmaceutique selon la revendication 13, dans laquelle la composition pharmaceutique est exempte de choline.

15. Composition pharmaceutique selon la revendication 13 ou 14, dans laquelle une dose unitaire de la composition comprend :
- 100 µg de vitamine B12 ; et
- 28 mg de fer.

16. Composition pharmaceutique selon l'une quelconque des revendications 13 à 15, dans laquelle une dose unitaire de ladite composition comprend en outre :
- entre 1 mg et 4 mg de vitamine B2 ;
- entre 12 mg et 50 mg de niacine ;
- entre 4,5 mg et 18 mg de vitamine B5 ;
- entre 1 mg et 4 mg de vitamine B6 ;
- entre 75 µg et 300 µg de biotine ;
- entre 50 mg et 200 mg de vitamine C ;
- entre 3,5 µg et 75 µg de vitamine D3 ;
- entre 6 mg et 24 mg de vitamine E ;
- entre 20 µg et 80 µg de chrome ;
- entre 75 µg et 300 µg d'iode ;
- entre 1,5 mg et 6 mg de manganèse ;
- entre 25 µg et 100 µg de molybdène ; et
- entre 27 µg et 110 µg de sélénium.

17. Composition pharmaceutique selon l'une quelconque des revendications 13, 15 ou 16, dans laquelle une dose unitaire de la composition comprend en outre de la choline en une quantité de 20 mg ou moins.

18. Composition pharmaceutique selon l'une quelconque des revendications 13 à 17, dans laquelle une dose unitaire de la composition comprend environ 75 microgrammes de vitamine D3.

19. Composition pharmaceutique selon l'une quelconque des revendications 13 à 18, dans laquelle une dose unitaire de la composition comprend environ 2 mg de vitamine B6.
